# EUROPEAN PATENT SPECIFICATION

(11) **EP 1 663 339 B1**
(45) Date of publication and mention of the grant of the patent: **31.07.2013**
(21) Application number: 04766458.6
(22) Date of filing: 09.08.2004
(51) Int. Cl.: A61L 31/16, A61L 29/16, A61F 2/06, A61K 31/4045, C07D 209/16, A61P 9/00, A61M 25/00

(54) **ENDOLUMINAL PROSTHESIS COMPRISING A THERAPEUTIC AGENT**
ENDOLUMINALE PROTHESE MIT EINEM THERAPEUTISCHEN MITTEL
PROTHESE ENDOLUMINALE COMPRENANT UN AGENT THERAPEUTIQUE

(30) Priority: 14.08.2003 EP 03447210
(43) Date of publication of application: 07.06.2006
(73) Proprietor: Blue Medical Devices B.V., 5708 HN Helmond (NL)
(72) Inventor: DE SCHEERDER, Ivan, Kamiel, B-3020 Herent (BE); HORVERS, Ronald, Adrianus, Maria, NL-5663 PH Geldrop (NL)
(74) Representative: Gyi, Jeffrey Ivan
(86) International application number: PCT/EP2004/051755
(87) International publication number: WO 2005/016400

(56) References cited:
- EP-A- 0 950 386
- WO-A-00/45809
- WO-A-01/49268
- WO-A-98/30255

## Description

### Field of the Invention

Delivery of a therapeutic agent locally, in particular from an intraluminal prosthesis such as a coronary stent, directly from the surface of the prosthesis or from pores, micropores, perforationsor pits in the prosthesis body, directly bounded on the prosthesis or mixed or bound to a polymer coating applied on the prosthesis, or mixed or bound to a glue applied to the prosthesis, to modulate the healing response after vascular injury, to improve endothelial cell regrowth, and to inhibit inflammation induced by the injury caused by the implantation of the intraluminal prosthesis and inhibiting tissue proliferation and thereby preventing stenosis of the prosthesis.

### Background of the Invention:

Re-narrowing (restenosis) of an atherosclerotic coronary artery after percutaneous transluminal coronary angioplasty (PTCA) occurs in 10-50% of patients undergoing this procedure and subsequently requires either further angioplasty or coronary artery bypass graft. While the exact hormonal and cellular processes promoting restenosis are still being determined, the present understanding is that the process of PTCA, besides opening the atherosclerotically obstructed artery, also injures resident coronary arterial smooth muscle cells (SMC). In response to this injury, adhering platelets, infiltrating macrophages, leukocytes, or the smooth muscle cells (SMC) themselves release cell derived growth factors with subsequent proliferation and migration of medial SMC through the internal elastic lamina to the area of the vessel intima. Further proliferation and hyperplasia of intimal SMC and, most significantly, production of large amounts of extracellular matrix over a period of 3-6 months results in the filling in and narrowing of the vascular space sufficient to significantly obstruct coronary blood flow.

Several recent experimental approaches to preventing SMC proliferation have shown promise although the mechanisms for most agents employed are still unclear. Heparin is the best known and characterised agent causing inhibition of SMC proliferation both in vitro and in animal models of balloon angioplasty-mediated injury. The mechanism of SMC inhibition with heparin is still not known but may be due to any or all of the following: 1) reduced expression of the growth regulatory protooncogenes c-fos and c-myc, 2) reduced cellular production of tissue plasminogen activator, or 3) binding and dequestration of growth regulatory factors such as fibrovalent growth factor (FGF).

Other agents which have demonstrated the ability to reduce myointimal thickening in animal models of balloon vascular injury are angiopeptin (a somatostatin analog), calcium channel blockers, angiotensin converting enzyme inhibitors (captopril, cilazapril), cyclosporin A, trapidil (an antianginal, antiplatelet agent), terbinafine (antifungal), colchicine and taxol (antitubulin antiproliferatives), and c-myc and c-myb antinsense oligonucleotides.

Additionally, a goat antibody to the SMC mitogen platelet derived growth factor (PDGF) has been shown to be effective in reducing myointimal thickening in a rat model of balloon angioplasty injury, thereby implicating PDGF directly in the etiology of restenosis. Thus, while no therapy has as yet proven successful clinically in preventing restenosis after angioplasty, the in vivo experimental success of several agents known to inhibit SMC growth suggests that these agents as a class have the capacity to prevent clinical restenosis and deserve careful evaluation in humans.

Coronary heart disease is the major cause of death in men over the age of 40 and in women over the age of fifty in the western world.

Most coronary artery-related deaths are due to atherosclerosis. Atherosclerotic lesions which limit or obstruct coronary blood flow are the major cause of ischemic heart disease related mortality and result in 500,000-600,000 deaths in the United States annually. To arrest the disease process and prevent the more advanced disease states in which the cardiac muscle itself is compromised, direct intervention has been employed via percutaneous transluminal coronary angioplasty (PTCA) or coronary artery bypass graft (CABG).

PTCA is a procedure in which a small balloon-tipped catheter is passed down a narrowed coronary artery and then expanded to re-open the artery. It is currently performed in approximately 250,000-300,000 patients each year. The major advantage of this therapy is that patients in which the procedure is successful need not undergo the more invasive surgical procedure of coronary artery bypass graft. A major difficulty with PTCA is the problem of post-angioplasty closure of the vessel, both immediately after PTCA (acute reocclusion) and in the long term (restenosis).

The mechanism of acute reocclusion appears to involve several factors and may result from vascular recoil with resultant closure of the artery and/or deposition of blood platelets along the damaged length of the newly opened blood vessel followed by formation of a fibrin/red blood cell thrombus. Recently, intravascular stents have been examined as a means of preventing acute reclosure after PTCA.

Restenosis (chronic reclosure) after angioplasty is a more gradual process than acute reocclusion: 30% of patients with subtotal lesions and 50% of patients with chronic total lesions will go on to restenosis after angioplasty. While the exact mechanism for restenosis is still under active investigation, the general aspects of the restenosis process have been identified:

In the normal arterial wall, smooth muscle cells (SMC) proliferate at a low rate (<0.1%/day). SMC in vessel wall exists in a 'contractile' phenotype characterised by 80-90% of the cell cytoplasmic volume occupied with the contractile apparatus. Endoplasmic reticulum, golgi bodies, and free ribosomes are few and located in the perinuclear region. Extracellular matrix surrounds SMC and is rich in heparin-like glycosylaminoglycans which are believed to be responsible for maintaining SMC in the contractile phenotypic state.

Upon pressure expansion of an intracoronary balloon catheter during angioplasty, smooth muscle cells within the arterial wall become injured. Cell derived growth factors such as platelet derived growth factor (PDGF), basic fibroblast growth factor (bFGF), epidermal growth factor (EGF), etc. are released from platelets (i.e., PDGF) adhering to the damaged arterial luminal surface, invading macrophages and/or leukocytes, or directly from SMC (i.e., BFGF) provoke a proliferation and migratory response in medial SMC. These cells undergo a phenotypic change from the contractile phenotype to a 'synthetic' phenotype characterised by only few contractile filament bundles but extensive rough endoplasmic reticulum, golgi and free ribosomes. Proliferation/migration usually begins within 1-2 days post-injury and peaks at 2 days in the media, rapidly declining thereafter (Campbell et al., In: Vascular Smooth Muscle Cells in Culture, Campbell, J.H. and Campbell, G.R., Eds, CRC Press, Boca Ration, 1987, pp. 39-55) ; Clowes, A.W. and Schwartz,. S.M., Circ. Res. 56:139-145, 1985).

Finally, daughter synthetic cells migrate to the intimal layer of arterial smooth muscle and continue to proliferate. Proliferation and migration continues until the damaged luminal endothelial layer regenerates at which time proliferation ceases within the intima, usually within 7-14 days post-injury. The remaining increase in intimal thickening which occurs over the next 3-6 months is due to an increase in extracellular matrix rather than cell number. Thus, SMC migration and proliferation is an acute response to vessel injury while intimal hyperplasia is a more chronic response. (Liu et al., Circulation, 79:1374-1387, 1989).

Patients with symptomatic reocclusion require either repeat PTCA or CABG. Because 30-50% of patients undergoing PTCA will experience restenosis, restenosis has clearly limited the success of PTCA as a therapeutic approach to coronary artery disease. Because SMC proliferation and migration are intimately involved with the pathophysiological response to arterial injury, prevention of SMC proliferation and migration represents a target for pharmacological intervention in the prevention of restenosis.

### Novel Features and Applications to Stent Technology

Currently, attempts to improve the clinical performance of endoluminal prosthesis such as coronary stents have involved some variation of either searching for a more biocompatible metal alloy, optimising the stent surface, applying a coating to the metal, attaching a covering or membrane, or embedding material on the surface via ion bombardment. A stent designed to include reservoirs that can be filled up with therapeutic agents, influencing the restenosis process has also been proposed.

### Local Drug Delivery from an endoluminal prosthesis such as a Stent to Inhibit Restenosis

In this application, a therapeutic agent is delivered to the site of arterial injury. The conventional approach has been to incorporate the therapeutic agent into a polymer material which is then coated on the stent. The ideal coating material must be able to adhere strongly to the metal stent both before and after expansion, be capable of retaining the drug at a sufficient load level to obtain the required dose, be able to release the drug in a controlled way over a period of several weeks, and be as thin as possible so as to minimize the increase in profile. In addition, the coating material should not contribute to any adverse response by the body and should be perfectly biocompatible (i.e., should be non-thrombogenic, non-inflammatory, etc.). To date, the ideal coating material has not been developed for this application.

An alternative to this polymer/drug loading method is direct binding of the therapeutic agent to the metal surface. This method has the advantage to be perfectly biocompatible. Disadvantages are however the limited dose of drug that can be loaded on the stent and the (too) fast release of the drug.

An other alternative is to use a drug impregnated biocompatible glue, in particular a biocompatible oil/solvent emulsion. Also with this method the drug release is quite fast, but combination with a barrier coating could improve the release characteristics.

Another approach is to design a stent that contains reservoirs which could be loaded with the drug. A coating or membrane of biocompatible material could be applied over the reservoirs which would control the diffusion of the drug from the reservoirs to the arterial wall. The advantages of this system is that much more drug can be loaded and much longer drug release can be achieved.

### Pharmacologic attemps to prevent restenosis

Pharmacological attempts to prevent restenosis by pharmacologic means have thus far been unsuccessful and all involve systemic administration of the trial agents. Neither aspirin-dipyridamole, ticlopidine, acute heparin administration, chronic warfarin (6 months) nor methylprednisolone have been effective in preventing restenosis although platelet inhibitors have been effective in preventing acute reocclusion after angioplasty. The calcium antagonists have also been unsuccessful in preventing restenosis, although they are still under study. Other agents currently under study include thromboxane inhibitors, prostacyclin mimetics, platelet membrane receptor blockers, thrombin inhibitors and angiotensin converting enzyme inhibitors. These agents must be given systemically, however, and attainment of a therapeutically effective dose may not be possible; antiproliferative (or anti-restenosis) concentrations may exceed the known toxic concentrations of these agents so that levels sufficient to produce smooth muscle inhibition may not be reached (Lang et al., 42 Ann. Rev. Med., 127-132 (1991); Popma et al., 84 Circulation, 1426-1436 (1991)).

Additional clinical trials in which the effectiveness for preventing restenosis of dietary fish oil supplements, thromboxane receptor antagonists, cholesterol lowering agents, and serotonin antagonists has been examined have shown either conflicting or negative results so that no pharmacological agents are as yet clinically available to prevent post-angioplasty restenosis (Franklin, S.M. and Faxon, D.P., 4 Coronary Artery Disease, 232-242 (1993); Serruys, P.W. et al., 88 Circulation, (part 1) 1588-1601, (1993).

Stents have proven useful in reducing restenosis. Stents, which when expanded within the lumen of an angioplastied coronary artery, provide structural support to the arterial wall, are helpful in maintaining an open path for blood flow. In two randomized clinical trials, stents were shown to increase angiographic success after PTCA, increased the stenosed blood vessel lumen and reduced the lesion recurrence at 6 months (Serruys et al., 331 New Eng Jour. Med, 495, (1994); Fischman et al., 331 New Eng Jour. Med, 496-501 (1994). Additionally, in a preliminary trial, heparin coated stents appear to possess the same benefit of reduction in stenosis diameter at follow-up as was observed with non-heparin coated stents. Additionally, heparin coating appears to have the added benefit of producing a reduction in sub-acute thrombosis after stent implantation (Serruys et al., 93 Circulation, 412-422, (1996). Thus, 1) sustained mechanical expansion of a stenosed coronary artery has been shown to provide some measure of restenosis prevention, and 2) coating of stents with heparin has demonstrated both the feasibility and the clinical usefulness of delivering drugs to local, injured tissue off the surface of the stent.

Numerous agents are being actively studied as anti proliferative agents for use in restenosis and have shown some activity in experimental animal models. These include: heparin and heparin fragments (Clowes and Kamovsky, 265 Nature, 25-626, (1977); Guyton, J.R. et al. 46 Circ. Res., 625-634, (1980); Clowes, A.W. and Clowes, M.M., 52 Lab. Invest., 611-616, (1985); Clowes, A.W. and Clowes, M.M., 58 Circ. Res., 839-845 (1986); Majesky et al., 61 Circ Res., 296-300, (1987); Snow et al., 137 Am. J. Pathol., 313-330 (1990); Okada, T. et al., 25 Neurosurgery, 92-898, (1989), colchicine (Currier, J.W. et al., 80 Circulation, 11-66, (1989), taxol, angiotensin converting enzyme (ACE)inhibitors (Powell, J.S. et al., 245 Science, 186-188 (1989), angiopeptin (Lundergan, C.F. et al., 17 Am. J. Cardiol. (Suppl. B); 132B-136B (1991), Cyclosporin A (Jonasson, L. et. al., 85 Proc. Nati, Acad. Sci., 2303 (1988), goat-anti-rabbit PDGF antibody (Ferns, G.A.A., et al., 253 Science, 1129-1132 (1991), terbinafine (Nemecek, G.M. et al., 248 J. Pharmacol. Exp. Thera., 1167-11747 (1989), trapidil (Liu, M.W. et al., 81 Circulation, 1089-1093 (1990), interferon-gamma (Hansson, G.K. and Holm, 84 J. Circulation, 1266-1272 (1991), steroids(Colbum, M.D. et al., 15 J. Vasc. Surg., 510-518 (1992), see also Berk, B.C. et al., 17 J. Am. Coll. Cardiol., 111B-1 17B (1991), ionizing radiation , fusion toxins, antisense oligonucleotides, gene vectors and rapamycin. The systematic administration of probucol for the inhibition of atherogenesis following balloon angioplasty has been demonstrated (Schneider et al.(1993) Circulation 88: 628-637, Tardiff et al. (1996) abstract 0524, Circulation 941-91, Rodés et al. Circulation 1998;97:429-436, Tardiff et al. Circulation 2003;107:552 and Lau et al. Circulation 2003;107:2031). The use of anti-oxidants and/or free-radical scavengers for inhibiting restenosis is described in U.S. 5,326,757; WO 95/26193; and CA 2106695.

WO 98/30255 discloses the use of probucol and of a large group of other antioxidant substances, including melationin, for inhibition of restenosis in recanalized blood vessels using a specially designed local drug delivery catheter. The use of this local drug delivery catheter is intended to reduce the total drug dosage required and to achieve much higher local concentrations than is possible with systemic delivery. However, although several studies have demonstrated that probucol is effective in reducing restenosis (see the above mentioned publications), any effect of a local delivery of probucol has not yet been demonstrated, even not in WO 98/30255 itself.

Rapamycin coated on a stent, using a mixture of rapamycin in a polymer solution has been described in EP-A-0 950 386. Clinical studies have also shown a dramatic decrease of the restenosis rates using a rapamycin coated stent. Potential disadvantages of this system is the use of rapamycin, which is a toxic drug that affects not only SMC proliferation, but also endothelial cell regrowth and restoration and fibroblast proliferation after stent implantation, and the use of a polymer which always leads to the concern of an inflammatory reaction induced by the polymer, and potentially occurrence of late restenosis.

### Summary of the Invention

Melatonin (N-acetyl-5-methoxyrtryptamine) coated on an endoluminal prosthesis to modulate the healing response after vascular injury by decreasing vascular injury and inflammation caused by the implantation of the prosthesis and resulting in a decreased neointimal hyperplasia.

The present application relates to the use of melatonin (N-acetyl-5-methoxytryptamine) for the manufacture of a releasable therapeutic agent comprised in a stent arranged to be implanted at least partially in a blood vessel in contact with a wall of the blood vessel , the therapeutic agent being present in an amount effective to modify the healing response of the vessel wall after tissue injury caused by the implantation of the stent by inhibiting inflammation, cell proliferation and cell ingrowth into the stent. In vitro evidence demonstrates that melatonin has a mode of action which is different from that of rapamycin. Melatonin has been shown to possess anti-inflammatory effects, among a number of other actions. Melatonin reduces tissue destruction during inflammatory reactions by a number of means. Melatonin, by virtue of its ability to directly scavenge toxic free radicals, reduces macromolecular damage in all organs. The free radicals and reactive oxygen and nitrogen species known to be scavaged by melatonin include highly toxic hydroxyl radicals (-OH), peroxynitrite anion (ONOO-), and hypochlorous acid (HOCL), among others. These agents all contribute to the inflammatory response and associated tissue destruction. Additionally, melatonin has other means to lower the damage resulting from inflammation. Melatonin prevents the translocation of nuclear factor-kappa B (NF-kappaB) to the nucleus and its binding to DNA, thereby reducing the upregulation of a variety of proinflammatory cytokines, for example, interleukins and tumor necrosis factor alpha. Finally, there is indirect evidence that melatonin inhibits the production of adhesion molecules that promote the sticking of leukocytes to endothelial cells. By this means melatonin attenuates transendothelial cell migration and edema, which contribute to tissue damage.

Although most free radical scavengers and anti-inflammatory agents are considered not potent enough to block in-stent neointimal hyperplasia sufficiently, the present inventor has found a significant inhibition of peri-strut injury, peri-strut inflammation and neointimal hyperplasia in a pig stent coronary model. He found an inhibition of the same magnitude as for potent antiproliferative drugs like sirolimus and paclitaxel using the same coating technology and using the same animal model suggesting that melatonin is equipotent compared to these potent antiproliferative drugs and is more potent than pure antioxidants like probucol. Antiproliferative drugs however are affecting all cells involved in the healing process, resulting in severe side effects. By blocking endothelial cell regrowth for example they will delay the recovery of the endothelial cells layer, resulting in a longer contact between the injured vascular wall with the circulating blood. This results in platelet activation, thrombus formation and the risk for stent trombosis and furthermore platelets and trombine are potent activators of smooth muscle cells resulting in a continuous stimulus for neointimal hyperplasia. The inhibitory effect on fibroblast is less well understood, however could be responsible for the frequent stent malappositioning found with rapamycin coated stents. The advantage of using melatonin compared to sirolimus and especially paclitaxel is that melatonin does not block cells that are involved in the healing response. It only neutralise toxic compounds that lead to further cell damage, inflammation and an overstimulation of the smooth muscle cells resulting in neointimal hyperplasia and restenosis. Melatonin neutralises toxic compounds released by inflammatory cells in response to vascular injury. These compounds are tought to be responsible for an overstimulation of the healing response, leading to an abundant neointimal proliferation and restenosis. Melatonin has also no direct effect on the endothelial cell regrowth and indirectly, by eliminating toxic substances that also are toxic for endothelial cells, a positive effect on endothelial cell regrowth. Therefore there is no problem with potential late thrombotic occlusion of the stent by thrombus formation. Another advantage compared to the use of antiproliferative medications is that melatonin is not cytotoxic for smooth muscle cells and other cells involved in the neointimal hyperplasia cascade, even at very high drug concentrations. Therefore, by using melatonin, toxic products that increase tissue damage and by doing so overstimulate the healing response resulting in an inappropriate smooth muscle cell proliferation, neointimal hyperplasia and finally resulting in stent narrowing are neutralised so that the stimulus for smooth muscle cell dedifferentiation and proliferation is eliminated before the neointimal hyperplasia cascade is stimulated. Different from other antioxidant drugs, melatonin has shown to have also direct effects on the inflammatory cells, inhibiting their activation during inflammatory processes after tissue injury. The importance of this pathway is not well understood, but seems to be crucial in the beneficial effects of local melatonin delivery on the healing response after vascular injury. Apart from these effects, our experimental results suggest also a direct inhibitory effect of melatonin on smooth muscle cell dedifferentiation and proliferation.

So far local delivery of melatonin was used in coated veterinary implants for regulation of seasonal breeding and other physiological responses. WO 98/30255 mentiones localised intravascular delivery of probucol, and of a series of other antioxidant substances including melatonin, in an amount sufficient to inhibit restenosis in recanalized blood vessels. Most commonly, use is made of a specially designed local drug delivery catheter but, in some cases, it may be advantageous according to WO 98/30255 to employ implanted devices, such as implanted stents capable of delivering the antioxidant substance for prolonged periods of time. Disadvantage of the use of a local drug delivery balloon is that the drug can only be released during a limited time period (up to 5 minutes) and that the efficacy of effective local drug delivery to the vascular wall is very low (<1%-5%) and very variable. So far no beneficial effect of using localized intravascular delivery of antioxidant substances like described in WO 98/30255 for the inhibition of restenosis in recanalized blood vessels, using a local drug delivery balloon nor a drug coated stent has been shown, especially even not in WO 98/30255 itself. For Melatonin, no proof is given in WO 98/30255 that melatonin may be effective in inhibiting restenosis. Instead, a reference is made for the antioxidant substances, different from probucol, to US 5 326 757; WO 95/26193 and CA 2 106 695 disclosing however specific combinations of selected antioxidant substances (no melatonin) or a combination of antioxidant substances (Vitamin C) with hyaluronic acid to enhance the effect of this hyaluronic acid in preventing narrowing of tubular walls. For probucol, which is according to WO 98/30255 the preferred antioxidant, reference is on the contrary made to publications demonstrating the effectiveness of probucol for inhibiting restenosis. Consequently, for a skilled person it is clear that in the some cases wherein it may be advantageous to use an implanted stent instead of a drug delivery catheter, the antioxidant substance should be the preferred probutol since it is clear that the amount of the antioxidant substance which can be delivered with an implanted stent is much smaller than the amount that can be delivered with a catheter and that much larger amounts of the other antioxidant substances will be required to inhibit restenosis than of the more effective probucol. WO 98/30255 thus does not teach the combination of an implanted stent coated with melatonin so that this combination is novel with respect to WO 98/30255. Based on the teachings of WO 98/30255 a skilled person could try to deliver probucol by means of an implanted stent but, if that does not give the desired results on inhibiting restenosis, it is not obvious for him to try also the other antioxidant substances which were for the skilled person apparently less effective in inhibiting restenosis than probucol.

The present invention is now based on the unexpected potent beneficial effect of local, stent mediated melatonin delivery on the vascular injury and inflammation, most probably due to the potent neutralizing effect of toxic free radicals, released during injury induced inflammation, by melatonin, combined with its direct anti-inflammatory effects, resulting in an improved healing, less smooth muscle cell stimulation and proliferation and less cellular ingrowth and narrowing of an endoluminal implant, endovascular prosthesis, shunt or catheter.

### Experimental work with melatonin (N-acetyl-5-methoxytryptamine) coated coronary stents:

To get rid of the polymer, which remains always a concern when coating a drug on an endoluminal prosthesis, since several polymers have shown to be non biocompatible and to induce an inflammatory response leading to SMC proliferation and restenosis, the present inventor tried to coat melatonin directly to the surface of the endoluminal prosthesis. As endoluminal prosthesis use was made of a commercially available 316L stainless steel coronary stent (V-Flex Plus, 16mm/3.0mm, William Cook Europe) and the stent was dipped in a 20mg/ml ethanol solution for 30 seconds. After removal the stent was air-dried using a warm laminar flow to evaporate the ethanol. Using this method a total melatonin load on the stent of 20 µg could be achieved. Implantation of these stents in a porcine coronary model with follow-up after 5 days revealed perfect biocompatibility of the system, without inducing any inflammation surrounding the struts of the stents on histological examination. After 4 weeks a 26% decrease in neointimal hyperplasia was surprisingly found compared to a bare stent. Similar experiments, using probucol, did not result in a significant effect on inflammatory response and neointimal hyperplasia. Notwithstanding the low total dose of melatonin (20µg), significant efficacy with this system could be demonstrated. This could be explained by the 1) the potent free radical scavenging effect of melatonin, 2) the potent anti-inflammatory effect of melatonin, 3) maybe a direct effect on SMC proliferation, 4) the non toxic effect of melatonin on other mediators of the healing response.

In a next step the total load of melatonin was increased by using more concentrated melatonin/ethanol solutions. By doing so a maximum total load of 300 µg/ stent could be achieved. Porcine experiments revealed an efficient blocking of inflammatory response and neointimal hyperplasia using this high dose melatonin loaded stents.

A disavantage of the direct coating system is the fast release of the drug from the stent. In-vitro release curves showed a 90% release within 24 hours. In-vivo studies however showed sufficient melatonin coronary vascular concentration up to 15 days.

In order to maintain significant melatonin concentrations for a longer period of time to obtain a durable anti-restenosis effect the present inventor has developed new methods to achieve a slower melatonin release. The drug was dissolved in a biocompatible emulsion of oil and a solvent wherein the drug is highly soluble. The stent was dipped in this emulsion several times and in between the different dipping steps air-dried using a warm laminar flow to evaporate the solvent and harden the drug/oil coating. This system resulted in a total melatonin load per stent of 500 µg and a much slower melatonin release over a time period of weeks instead of days.

In-vivo work in a porcine coronary model demonstrated the perfect biocompatibility of this coating method, since no inflammation was evoked at 5 days follow-up using non drug loaded stents.

Sustained inhibition of peri-strut inflammation and neointimal hyperplasia was seen up to 3 months using stents loaded with 500 µg of melatonin using this method. Using the same coating method, no effect of probucol was found.

### Comparative tests: Pre-clinical evaluation of Probucol and Melatonin loaded stents.

### Stent Implantation

Domestic crossbred pigs of both sexes weighing 20-25kg were used. They were fed with a standard natural grain diet without lipid or cholesterol supplementation throughout the study. All animals were treated and cared for in accordance with the Belgium National Institute of Health Guidelines for care and use of laboratory animals.

In this study, bare Jostent Flexmaster stents (n=8), Bare stents dipped in a Biological oil coating (Oil Only; n=7), Bare stents dipped in a Probucol/Biological oil coating solution resulting in a total Probucol load of 447+/-56µg/per stent (Pro, n=7) and Bare stents dipped in a Melatinin/Biological oil coating solution resulting in a total Melatonin load of 367+/47µg/stent (Mela, n=8). Stents were implanted in the coronary arteries of 15 pigs. All pigs were followed for 4 weeks to evaluate the peri-strut inflammatory response and neointimal formation.

Surgical procedure and stent implantation in the coronary arteries were performed according to the method described by De Scheerder et al. in "Local angiopeptin delivery using coated stents reduces neointimal proliferation in overstretched porcine coronary arteries." J. Inves. Cardiol. 8:215-222; 1996, and in "Experimental study of thrombogenicity and foreign body reaction induced by heparin-coated coronary stents." Circulation 95:1549-1553; 1997. The guiding catheter was used as a reference to obtain an oversizing from 10 to 20%.

### Methods

### Histopathotogy

Coronary segments were carefully dissected together with a 1 cm minimum vessel segment both proximal and distal to the stent. The segments were fixed in a 10% formalin solution. The middle part of each stent was harvested for histopathologic analysis. Tissue specimens were embedded in a cold-polymerizing resin (Technovit 7100, Heraus Kulzer GmbH, and Wehrheim, Germany). Sections, 5 microns thick, were cut with a rotary heavy duty microtome HM 360 (Microm, Walldorf, Germany) equipped with a hard metal knife and stained with hematoxylin-eosin, elastic stain and phosphotungstic acid hematoxylin stain. Light microscopic examination was performed by an experienced pathologist who was blinded to the type of stent used. Injury of the arterial wall due to stent deployment (and eventually inflammation induced by the polymer) was evaluated for each stent filament and graded as described by Schwartz et al.
Grade 0 = internal elastic membrane intact, media compressed but not lacerated;
Grade 1 = internal elastic membrane lacerated;
Grade 2 = media visibly lacerated; external elastic membrane compressed but intact;
Grade 3 = large laceration of the media extending through the external elastic membrane or stent filament residing in the adventitia.

Inflammatory reaction at each stent filament was carefully examined, searching for inflammatory cells, and scored as followed:
1 = sparsely located histolymphocytes surrounding the stent filament;
2 = more densely located histolymphocytes covering the stent filament, but no lymphogranuloma and/or giant cells formation found;
3 = diffusely located histolymphocytes, lymphogranuloma and/or giant cells, also invading the media.
Mean score = sum of score for each filament/ number of filament present.

### Results

### Stent implantation

All stents were implanted successfully. Angiography after stent implantation showed arterial lumen patency. All pigs were controlled at the planned time.

### Histopathoiogy

Stent struts were well aligned to arterial wall. Media layer was minimal to moderate compressed. Increased arterial injury was noted in bare and oil-only stent groups. Internal elastic lamina and media layer laceration were observed. A few stent struts showed a lacerated external elastic lamina. The arterial injury of Probutol and Melatonin groups were low. Especially the Melatonin stent group, only internal elastic lamina laceration was found. Compared to the oil-only stent group, the injury score was dramatically decreased.

Increased and more variable peri-strut inflammation was noted in the bare and oil-only groups. In some sections, occasional inflammatory cells were observed around the stent struts. However in some section, severe inflammation scored up to 3 was present in some stent struts. In Probutol stent group, a few stent struts had increased inflammatory response scored as 2. Furthermore, peri-strut hemorrhage was found in some sections. Melatonin stents showed a minimal inflammatory response. Both the inflammation scores of Probutol and Melatonin stent groups were lower than bare and oil-only stent groups (Probutol 1.03±0.08; Melatonin 1.00±0.00 vs bare 1.18±0.40; oil-only 1.21±0.49). No polymer residual was observed around stent struts in all coated groups.

### Morphometry (Table)

The neointimal hyperplasia of all groups was well organized. The lumen area of Melatonin stents was larger than the other groups. Furthermore the neointimal hyperplasia (0.89±0.28 mm2) and area stenosis (16±7%) of the Melatonin group were very limited, although the oversizing (balloon-area/IEL-area) was comparable among groups.

**Table. Histomorphometric evaluation of Probucol and Melatonin loaded stents at 4 weeks follow-up**

| | **N** | **LA (mm2)** | **NIH(mm2)** | **AS(%)** | **Bal/IEL** | **Injury** | **Inflammation** |
|---|---|---|---|---|---|---|---|
| Bare | 8 | 4.89±1.54 | 1.84±0.86 | 28±15 | 1.26±0.16 | 0.42±0.51 | 1.18±0.40 |
| | | | | | | | |
| Oil-only | 7 | 4.45±2.18 | 1.54±0.97 | 29±24 | 1.47±0.25 | 0.50±0.46 | 1.21±0.49 |
| Probucol | 7 | 4.46±1.06 | 1.43±0.77 | 24±14 | 1.38±0.10 | 0.28±0.20 | 1.03±0.08 |
| Melatonin | 8 | 5.10±1.40 | 0.89±0.28 | 16±7 | 1.41±0.11 | 0.08±0.09 | 1.00±0.00 |

| | | | | | | | |
|---|---|---|---|---|---|---|---|
| N: number of stents LA: Lumen area IEL: area inside the internal elastic lamina NIH: Neointimal hyperplasia ( = IEL- LA) AS: Area stenosis ( = 100 * (1 - LA/IEL) Bal/IEL: Balloon-area/IEL-area | | | | | | | |

### Conclusions

Variable inflammation and neointimal hyperplasia were observed in oil-only stents. However, compared to the bare stents, no increased inflammatory response and neointimal hyperplasia were found.

Probucol loaded stents could decrease arterial injury and inflammatory response compared to the bare and oil-only stents, however no significant effect on neointimal hyperplasia was found.

Melatonin loaded stents resulted in a significant decrease of the inflammatory response and neointimal hyperplasia compared to bare and oil-only stents.

Further studies using different Probucol total loading doses per stent (10-2000µg) resulted in similar results showing no significant effect on in-stent neointimal hyperplasia, on the contrarary, Melatonin showed a dose depending response with a significant inhibition on neointimal hyperplasia which was already observed for a dose of 0.5 µg/mm² stent area (i.e. the surface area of the stent which is covered by the coating) suggesting again that melatonin has an inhibitory effect on in-stent neointimal hyperplasia not depending on its antioxidant properties.

### Indications

Local delivery of melatonin by coating melatonin onto an endoluminal prosthesis, shunt or catheter and local delivery of melatonin to the surrounding tissue after implantation of the prosthesis, shunt or catheter, resulting in an inhibition of tissue injury, inflammation and cell proliferation to prevent neointimal hyperplasia and restenosis, prevention of tumor expansion and ingrowth into an endoluminal prosthesis and prevention of ingrowth of tissue into catheters and shunts inducing their failure.

### Different Potential delivery methods for melatonin (N-acetyl-5-methoxytryptamine):

Local delivery of melatonin, analogs or therapeutic substances with similar working mechanisms from an endovascular prosthesis, catheter or shunt, from the struts of a stent, from perforations in the struts of the stents, from channels in the strut of the stent, from a hollow wire forming the stent, from a stent graft, grafts, stent cover or sheath.

Involving direct binding of the drug to the stent strut metal backbone and to the perforations, channels in the struts;
or involving a co-mixture with polymers (both degradable and nondegrading)or a biocompatible glue (in particular an oil or fat) to hold the drug to the stent or graft;
or entrapping the drug into the metal of the stent or graft body which has been modified to contain micropores, channels or perforations;
or including covalent binding of the drug to the stent via solution chemistry techniques or dry chemistry techniques (e.g. vapour deposition methods such as rf-plasma polymerization) and combinations thereof.

Extravascular delivery by the pericardial route.

Extravascular delivery by the advential application of sustained release formulations.

### 1. Direct drug coating on the metallic surface:

Stents are dipped in a solution of melatonin in a solvent, for example ethanol, at final concentration range 0.001 to 50 weight %. Solvent is allowed to evaporate to leave a film of melatonin on the stent.

### 2. Delivery from Polymer Matrix:

Solution of Melatonin, prepared in a solvent miscible with polymer carrier solution, is mixed with solution of polymer at final concentration range 0.001 weight % to 50 weight % of drug. Polymers are biocompatible (i.e., not elicit any negative tissue reaction or promote mural thrombus formation) and degradable, such as lactone-based polyesters or copolyesters, e.g., polylactide, polycaprolactonglycolide,polyorthoesters, polyanhydrides; poly-aminoacids; polysaccharides; polyphosphazenes; poly(ether-ester) copolymers, e.g., PEO-PLLA, or blends thereof. Nonabsorbable biocompatible polymers are also suitable candidates. Polymers such as polydimethylsiloxane; poly(ethylene-vingylacetate); acrylate based polymers or copolymers, e.g., poly(hydroxyethyl methylmethacrylate, polyvinyl pyrrolidinone; fluorinated polymers such as polytetrafluoroethylene; cellulose esters.

Polymer/drug mixture is applied to the surfaces of the stent by either dip-coating, or spray coating, or brush coating or dip/spin coating or combinations thereof, and the. solvent allowed to evaporate to leave a film with entrapped Melatonin.

### 3. Delivery from a biocompatible glue (oil/solvent emulsion).

Solution of Melatonin, mixed in an oil/solvent emulsion at final concentration range 0.001 weight % to 50 weight % of drug. Emulsion/drug mixture is applied to the surface of the stent by either dip coating, or spray coating, or brush coating or dip/ spin coating or combinations thereof, and the solvent is allowed to evaporate to leave a film of oil or fat with entrapped Melatonin.

### 4. Delivery from microporous depots, pores or perforations in stent backbone through either a Polymer Membrane Coating or Glue/drug coaling:

A stent, whose body has been modified to contain micropores, pores, channels, perforations or pits is dipped into a solution of Melatonin, range 0.001 wt% to saturated, in organic solvent such as acetone or methylene chloride, for sufficient time to allow solution to permeate into the pores. (The dipping solution can also be pressurised to improve the loading efficiency.) After the solvent has been allowed to evaporate, the stent is dipped briefly in fresh solvent to remove excess surface bound drug. Additionally a solution of polymer, chosen from any identified in the first experimental method, can be applied to the stent as detailed above. This outer layer of polymer will than act as release and diffusion-controller for release of drug.

### 5. Delivery via lysis of a Covalent Drug Tether

Melatonin is modified to contain a hydrolytically or enzymatically labile covalent bond for attaching to the surface of the stent which itself has been chemically derivatized to allow covalent immobilization. Covalent bonds such as ester, amides or anhydrides may be suitable for this.

### 6. Pericardial Delivery

A: Polymeric Sheet: melatonin is combined at concentration range previously highlighted, with a degradable polymer such as poly(caprolactone-glycolide) or non-degradable polymer, e.g., polydimethylsiloxane, and mixture cast as a thin sheet, thickness range 10p to 10 µm. The resulting sheet can be wrapped perivascularly on the target vessel. Preference would be for the absorbable polymer.

B: Conformal coating: Melatonin is combined with a polymer that has a melting temperature higher than 37°C, more particularly in the range of 40 to 45°C. Mixture is applied in a molten state to the external side of the target vessel. Upon cooling to body temperature the mixture solidifies conformally to the vessel wall. Both non-degradable and absorbable biocompatible polymers are suitable.

## Claims

1. Use of melatonin (N-acetyl-5-methoxytryptamine) for the manufacture of a releasable therapeutic agent comprised in a stent arranged to be implanted at least partially in a blood vessel in contact with a wall of the blood vessel , the therapeutic agent being present in an amount effective to modify the healing response of the vessel wall after tissue injury caused by the implantation of the stent by inhibiting inflammation, cell proliferation and cell ingrowth into the stent.

2. Use according to claim 1, **characterised in that** said therapeutic agent is coated on the stent.

3. Use according to claim 1 or 2, **characterised in that** said stent is an endovascular stent, more particularly a coronary stent.

4. Use according to any of claims 1 to 3, **characterised in that** the stent is made of a wire, optionally a hollow wire filled with said therapeutic agent or with a product containing said therapeutic agent.

5. Use according to any of claims 1 to 3, **characterised in that** the stent comprises a generally thin walled cylinder, said cylinder containing a plurality of struts, said struts expandable depending on the amount of force applied to said strut, and said struts having a generally uniform thickness.

6. Use according to any of claims 1 to 3, **characterised in that** the stent comprises a generally thin walled structure containing a plurality of struts, the struts expandable to assume the shape of a lumen into which the stent is to be placed, said struts having a thickness and are provided with one or more recesses formed in at least one of said struts, said recesses having a closed perimeter on all sides and an open top and eventually an open bottom, the recesses containing said therapeutic agent or a product containing said therapeutic agent.

7. Use according to any one of the claims 1 to 6, **characterised in that** said melatonin, is coated either as such on the stent surface or is embedded in a biocompatible oil or fat or in a biocompatible polymer coated on the stent, or is conjugated to any substance coated on the stent.

8. Use according to any one of the claims 1 to 7, **characterised in that** the stent has a total load of said melatonin of at least 0. 001 micrograms/mm², preferably of at least 0.1 micrograms /mm², more preferably of at least 0.5 micrograms /mm² and most preferably of at least 2 micrograms /mm² stent area, the total load of said melatonin being preferably lower than 50 micrograms /mm², more preferably lower than 10 micrograms /mm², and most preferably lower than 6 micrograms /mm² stent area.

9. Use according to any one of the claims 1 to 8, **characterised in that** the stent is arranged to release said therapeutic agent over a period of at least 6 hours, preferably over a period of at least one week, after implantation in the blood vessel.

10. Use of Melatonin (N-acetyl-5-methoxytryptamine), as a single bioactive component or in combination with one or more other bioactive components, to manufacture a pharmaceutical composition for modifying the healing response after tissue injury caused by implantation or insertion of an endoluminal stent in a blood vessel, which melatonin is applied locally by coating melatonin from on a stent, which melatonin inhibits inflammation induced by the injury and prevents cell proliferation and cell ingrowth into the stent.

11. Use of melatonin (N-acetyl-5-methoxytryptamine), as a single bioactive component or in combination with one or more other bioactive components, to manufacture a pharmaceutical composition for inhibiting proliferation and hyperplasia of intimal smooth muscle cells after tissue injury caused by implantation or insertion of a stent.

12. Use according to claim 10 or 11, **characterised in that** said melatonin is applied on the surface of and/or in recesses in the stent.

## Patentansprüche

1. Verwendung von Melatonin (N-Acetyl-5-methoxytryptamin) für die Herstellung eines freisetzbaren therapeutischen Wirkstoffes enthalten in einem Stent, der derart angeordnet ist, dass er mindestens teilweise in ein Blutgefäß implantiert werden kann, in Kontakt mit einer Wand des Blutgefäßes, wobei der therapeutische Wirkstoff in einer Menge vorliegt, die wirksam für das Modifizieren der Heilungsreaktion der Gefäßwand nach einer Gewebeverletzung verursacht durch die Implantierung des Stents durch Verhinderung von Entzündung, Zellproliferation und Zelleinwachsen in den Stent ist.

2. Verwendung gemäß Anspruch 1, **dadurch gekennzeichnet, dass** der therapeutische Wirkstoff auf den Stent beschichtet ist.

3. Verwendung gemäß Anspruch 1 oder 2, **dadurch gekennzeichnet, dass** der Stent ein endovaskulärer Stent, insbesondere ein koronarer Stent ist.

4. Verwendung gemäß eines der Ansprüche 1 bis 3, **dadurch gekennzeichnet, dass** der Stent aus einem Draht hergestellt ist, wahlweise einem Hohldraht gefüllt mit dem therapeutischen Wirkstoff oder mit einem Produkt, welches den therapeutischen Wirkstoff enthält.

5. Verwendung gemäß eines der Ansprüche 1 bis 3, **dadurch gekennzeichnet, dass** der Stent einen im Allgemeinen dünnwandigen Zylinder umfasst, wobei der Zylinder mehrere Streben enthält, wobei die Streben abhängig von der auf die Streben angewandten Kraft ausdehnbar sind, und wobei die Streben eine im Allgemeinen gleichmäßige Dicke aufweisen.

6. Verwendung gemäß eines der Ansprüche 1 bis 3, **dadurch gekennzeichnet, dass** der Stent eine im Allgemeinen dünnwandige Struktur umfasst, die mehrere Streben enthält, wobei die Streben ausdehnbar sind, um die Form eines Lumens anzunehmen, in welches der Stent positioniert werden soll, wobei die Streben eine Dicke aufweisen und mit einer oder mehreren Aussparungen gebildet in mindestens einer der Streben versehen sind, wobei die Aussparungen einen geschlossenen Umfang an allen Seiten und eine offene Oberseite und letztendlich eine offene Unterseite aufweisen, wobei die Aussparungen den therapeutischen Wirkstoff oder ein Produkt, welches den therapeutischen Wirkstoff enthält, enthalten.

7. Verwendung gemäß eines der Ansprüche 1 bis 6, **dadurch gekennzeichnet, dass** das Melatonin entweder als solches auf die Stentoberfläche beschichtet ist oder in ein biokompatibles Öl oder Fett oder in ein biokompatibles Polymer beschichtet auf den Stent eingebettet ist oder zu jeglicher Substanz beschichtet auf den Stent konjugiert ist.

8. Verwendung gemäß eines der Ansprüche 1 bis 7, **dadurch gekennzeichnet, dass** der Stent eine Gesamtbeladung des Melatonins von mindestens 0,001 Mikrogramm/mm², vorzugsweise mindestens 0,1 Mikrogramm/mm², bevorzugter mindestens 0,5 Mikrogramm/mm² und am bevorzugtesten mindestens 2 Mikrogramm/mm² Stentbereich aufweist, wobei die Gesamtbeladung des Melatonins vorzugsweise geringer als 50 Mikrogramm/mm², bevorzugter geringer als 10 Mikrogramm/mm² und am bevorzugtesten geringer als 6 Mikrogramm/mm² Stentbereich ist.

9. Verwendung gemäß eines der Ansprüche 1 bis 8, **dadurch gekennzeichnet, dass** der Stent derart angeordnet ist, dass er den therapeutischen Wirkstoff über einen Zeitraum von mindestens 6 Stunden, vorzugsweise über einen Zeitraum von mindestens einer Woche nach der Implantierung in das Blutgefäß freisetzt.

10. Verwendung von Melatonin (N-Acetyl-5-methoxytryptamin) als eine einzelne bioaktive Komponente oder in Kombination mit einer oder mehreren anderen bioaktiven Komponenten zur Herstellung einer pharmazeutischen Zusammensetzung zum Modifizieren der Heilungsreaktion nach einer Gewebeverletzung verursacht durch die Implantierung oder Einführung eines endoluminalen Stents in ein Blutgefäß, wobei das Melatonin lokal durch Beschichtung des Melatonins auf einen Stent angewandt wird, wobei das Melatonin eine Entzündung induziert durch die Verletzung hemmt und die Zellproliferation und das Zelleinwachsen in den Stent verhindert.

11. Verwendung von Melatonin (N-Acetyl-5-methoxytryptamin) als eine einzelne bioaktive Komponente oder in Kombination mit einer oder mehreren anderen bioaktiven Komponenten zur Herstellung einer pharmazeutischen Zusammensetzung zur Hemmung der Proliferation und Hyperplasie intimaler glatter Muskelzellen nach einer Gewebeverletzung verursacht durch die Implantierung oder Einführung eines Stents.

12. Verwendung gemäß Anspruch 10 oder 11, **dadurch gekennzeichnet, dass** das Melatonin auf die Oberfläche des Stents und/oder in Aussparungen in dem Stent aufgetragen wird.

## Revendications

1. Utilisation de mélatonine (N-acétyle-5-méthoxytryptamine) pour la fabrication d'un agent thérapeutique libérable compris dans un stent configuré pour être implanté au moins partiellement dans un vaisseau sanguin en contact avec une paroi du vaisseau sanguin, l'agent thérapeutique étant présent en une quantité efficace pour modifier la réponse de cicatrisation de la paroi vasculaire, après une lésion du tissu résultant de l'implantation du stent, par inhibition de l'inflammation, de la prolifération cellulaire et de la croissance cellulaire dans le stent.

2. Utilisation selon la revendication 1, **caractérisée en ce que** ledit agent thérapeutique est revêtu sur le stent.

3. Utilisation selon la revendication 1 ou 2, **caractérisée en ce que** ledit stent est un stent endovasculaire, plus particulièrement un stent coronaire.

4. Utilisation selon l'une quelconque des revendications 1 à 3, **caractérisée en ce que** le stent est constitué d'un fil métallique, facultativement d'un fil métallique creux rempli dudit agent thérapeutique ou d'un produit contenant ledit agent thérapeutique.

5. Utilisation selon l'une quelconque des revendications 1 à 3, **caractérisée en ce que** le stent comprend un cylindre à parois généralement fines, ledit cylindre contenant une pluralité d'entretoises, lesdites entretoises étant extensibles en fonction de la quantité de force appliquée à ladite entretoise, et lesdites entretoises ayant une épaisseur généralement uniforme.

6. Utilisation selon l'une quelconque des revendications 1 à 3, **caractérisée en ce que** le stent comprend une structure à parois généralement fines contenant une pluralité d'entretoises, les entretoises étant extensibles pour prendre la forme d'une lumière dans laquelle le stent doit être placé, lesdites entretoises ayant une épaisseur et étant dotées d'une ou de plusieurs cavités formées dans au moins l'une desdites entretoises, lesdites cavités ayant un périmètre fermé sur tous les côtés, un haut ouvert et éventuellement un bas ouvert, les cavités contenant ledit agent thérapeutique ou un produit contenant ledit agent thérapeutique.

7. Utilisation selon l'une quelconque des revendications 1 à 6, **caractérisée en ce que** ladite mélatonine, est revêtue en tant que telle sur la surface du stent ou est intégrée dans une huile ou une graisse biocompatible ou dans un polymère biocompatible revêtu sur le stent, ou est conjuguée à toute substance revêtue sur le stent.

8. Utilisation selon l'une quelconque des revendications 1 à 7, **caractérisée en ce que** le stent a une charge totale de ladite mélatonine d'au moins 0,001 microgrammes/mm², de préférence d'au moins 0,1 microgrammes/mm², de manière davantage préférée d'au moins 0,5 microgrammes/mm² et idéalement d'au moins 2 microgrammes/mm² d'aire de stent, la charge totale de ladite mélatonine étant de préférence inférieure à 50 microgrammes/mm², de manière davantage préférée inférieure à 10 microgrammes/mm², et idéalement inférieure à 6 microgrammes/mm² d'aire de stent.

9. Utilisation selon l'une quelconque des revendications 1 à 8, **caractérisée en ce que** le stent est configuré pour libérer ledit agent thérapeutique sur une période d'au moins 6 heures, de préférence sur une période d'au moins une semaine, après l'implantation dans le vaisseau sanguin.

10. Utilisation de mélatonine (N-acétyl-5-méthoxytryptamine), en tant qu'agent bioactif unique ou en combinaison avec un ou plusieurs autres composants bioactifs, pour fabriquer une composition pharmaceutique destinée à modifier la réponse de cicatrisation après une lésion d'un tissu provoquée par l'implantation ou l'insertion d'un stent endoluminal dans un vaisseau sanguin, ladite mélatonine étant appliqués localement par revêtement de mélatonine sur un stent, ladite mélatonine inhibant l'inflammation induite par la lésion et empêchant la prolifération cellulaire et la croissance cellulaire dans le stent.

11. Utilisation de mélatonine (N-acétyl-5-méthoxytryptamine), en tant qu'agent bioactif unique ou en combinaison avec un ou plusieurs autres composants bioactifs, pour fabriquer une composition pharmaceutique destinée à inhiber la prolifération et l'hyperplasie des cellules musculaires lisses intimales après une lésion d'un tissu résultant de l'implantation ou de l'insertion d'un stent.

12. Utilisation selon la revendication 10 ou 11, **caractérisée en ce que** ladite mélatonine est appliquée sur la surface du stent et/ou dans des cavités dans le stent.
